# EUROPEAN PATENT APPLICATION

(11) **EP 4 706 562 A1**
(43) Date of publication of application: **11.03.2026**
(21) Application number: 24858022.7
(22) Date of filing: 28.06.2024
(51) Int. Cl.: A61B 17/32

(54) **MEDICAL ULTRASONIC SCALPEL, MEDICAL ULTRASONIC SCALPEL SYSTEM, AND ROBOT-ASSISTED ULTRASONIC SCALPEL SYSTEM**

(30) Priority: 28.08.2023 CN 202311094101
(71) Applicant: SMTP Medical Co., Ltd., Beijing 102629 (CN)
(72) Inventor: CAO, Qun, Beijing 102629 (CN); ZHAN, Songtao, Beijing 102629 (CN); FANG, Yingfei, Beijing 102629 (CN); DAI, Zhiling, Beijing 102629 (CN)
(74) Representative: MFG Patentanwälte Meyer-Wildhagen Meggle-Freund Gerhard PartG mbB
(86) International application number: PCT/CN2024/102391
(87) International publication number: WO 2025/044478

(57) **Abstract**

The present application relates to the technical field of medical devices, and provides a medical ultrasonic scalpel, a medical ultrasonic scalpel system, and a robot-assisted ultrasonic scalpel system. The medical ultrasonic scalpel includes a liquid flow sleeve (100) and a blade shank (200). The liquid flow sleeve (100) includes a sleeve body (101) and a first protruding part (102). The sleeve body (101) is provided with a mounting cavity (111) running through two opposite ends of the sleeve body (101), and the first protruding part (102) is located on an inner side wall of the sleeve body (101). The blade shank (200) runs through the mounting cavity (111), the blade shank (200) is pivotally connected to the liquid flow sleeve (100), the blade shank (200) and the liquid flow sleeve (100) are movable in an extension direction X, and an outer side wall of the blade shank (200) is provided with a second protruding part (202). When the liquid flow sleeve (100) rotates in a first rotation direction, the first protruding part (102) and the second protruding part (202) are offset from each other, so as to enable the liquid flow sleeve (100) to move in the extension direction X. When the liquid flow sleeve (100) rotates in a second rotation direction opposite to the first rotation direction, the first protruding part (102) abuts against the second protruding part (202), so as to drive the blade shank (200) to rotate in the second rotation direction. The medical ultrasonic scalpel implements the function of a wrench by means of the liquid flow sleeve, and reduces the number of tools used during assembly of the medical ultrasonic scalpel.

## Description

### CROSS REFERENCE

The present application refers to Chinese Patent Application No. 202311094101.5, filed on August 28, 2023 and entitled "MEDICAL ULTRASONIC SCALPEL, MEDICAL ULTRASONIC SCALPEL SYSTEM, AND ROBOT-ASSISTED ULTRASONIC SCALPEL SYSTEM", which is incorporated herein by reference in its entirety.

### TECHNICAL FIELD

The present application relates to the technical field of medical devices, and in particular to a medical ultrasonic scalpel, a medical ultrasonic scalpel system and a robot-assisted ultrasonic scalpel system.

### BACKGROUND ART

With the development of ultrasonic technology and its integration with modern medicine, medical ultrasonic scalpels have gradually been applied in surgical operations. For example, a medical ultrasonic scalpel can be used to perform cutting, drilling, or grinding of bone tissues, thereby achieving the purpose of treating the bone tissues.

During surgery, a medical ultrasonic scalpel will generate friction with body tissues, thereby generating heat. In some surgeries, the heat generated by friction will damage cellular tissues, cause postoperative complications, and produce adverse effects. Therefore, when using the medical ultrasonic scalpel for surgery, a liquid flow system is typically provided to cool friction hotspots on a blade shank by means of a liquid flow sleeve.

In the related art, the liquid flow sleeve and the blade shank are assembled on a handle by means of a wrench. The structure of the wrench is complex, and the assembly operation is inconvenient.

### SUMMARY

The present application is intended to solve at least one of the technical problems existing in the background art. To this end, it is an objective of the present application to provide a medical ultrasonic scalpel, a medical ultrasonic scalpel system and a robot-assisted ultrasonic scalpel system, in order to alleviate the problem of inconvenient assembly operations in the related art.

In a first aspect, an embodiment of the present application provides a medical ultrasonic scalpel, including: a liquid flow sleeve, including a sleeve body and a first protruding part, wherein the sleeve body is provided with a mounting cavity running through two opposite ends of the sleeve body, and the first protruding part is located on an inner side wall of the sleeve body; and a blade shank, wherein an extension direction of the blade shank is the same as an extension direction X of the liquid flow sleeve, the blade shank runs through the mounting cavity, the blade shank is pivotally connected to the liquid flow sleeve, the blade shank and the liquid flow sleeve are movable in the extension direction X, and an outer side wall of the blade shank is provided with a second protruding part, where the first protruding part and the second protruding part are configured to cooperate such that when the liquid flow sleeve rotates in a first rotation direction, the first protruding part and the second protruding part are offset from each other, so as to enable the liquid flow sleeve to move in the extension direction X, and when the liquid flow sleeve rotates in a second rotation direction opposite to the first rotation direction, the first protruding part abuts against the second protruding part, so as to drive the blade shank to rotate in the second rotation direction.

In some embodiments, the sleeve body is provided with at least one first mounting hole running through a side wall of the sleeve body; and the first protruding part includes a first portion and a second portion connected to each other, the first portion is located in the first mounting hole and connected to the sleeve body, the second portion is located in the mounting cavity, and the second portion and the second protruding part are configured such that, when the liquid flow sleeve rotates in the first rotation direction, the second protruding part pushes the second portion to move into the first mounting hole, and when the liquid flow sleeve rotates in the second rotation direction, the second portion abuts against the second protruding part.

In some embodiments, the second portion includes a first surface and a second surface, the first surface and the second surface being both parallel to the extension direction X, where the first surface is configured such that, when a thrust applied to the first surface toward the second surface is greater than a first preset thrust value, the second portion is moved into the first mounting hole, and the second surface is configured such that, when a thrust applied to the second surface toward the first surface is greater than a second preset thrust value, the liquid flow sleeve is moved.

In some embodiments, the second protruding part includes a third surface and a fourth surface, the third surface and the fourth surface being both parallel to the extension direction X; when the liquid flow sleeve rotates in the first rotation direction, the third surface is configured to apply a thrust to the first surface toward the second surface; and when the liquid flow sleeve rotates in the second rotation direction, the fourth surface is configured to apply a thrust to the second surface toward the first surface.

In some embodiments, a central axis of rotation of the liquid flow sleeve is located on a plane where the second surface is located, and the central axis of rotation of the liquid flow sleeve is offset from a plane where the first surface is located.

In some embodiments, the sleeve body is provided with two first mounting holes, the two first mounting holes being symmetrically spaced apart in a circumferential direction of the side wall of the sleeve body, and the liquid flow sleeve includes two first protruding parts in a one-to-one correspondence with the two first mounting holes.

In some embodiments, the liquid flow sleeve further includes: a protrusion connected to an outer side wall of the sleeve body, the protrusion being arranged spaced apart from the first mounting hole.

In some embodiments, the sleeve body is provided with a first mounting part, and the blade shank is provided with a second mounting part connected to the first mounting part.

In some embodiments, the first mounting part includes a first thread located on the side wall of the sleeve body, and the second mounting part includes a second thread located on a side wall of the blade shank, the second thread being threadedly connected to the first thread.

In some embodiments, the sleeve body is provided with at least one second mounting hole extending through the side wall of the sleeve body, and the first mounting part includes an elastic piece and a limiting protrusion connected to each other, the elastic piece being located in the second mounting hole, and the limiting protrusion being located in the mounting cavity; and the second mounting part includes a boss located on an outer side wall surface of the blade shank, the boss abutting against the limiting protrusion.

In some embodiments, one end of the blade shank is provided with a third mounting part, one end of the sleeve body is provided with a fourth mounting part, and the medical ultrasonic scalpel further includes: a handle, where the handle is provided with a fifth mounting part connected to the third mounting part, and a sixth mounting part connected to the fourth mounting part, the fifth mounting part and the sixth mounting part being both located at an end of the handle facing the blade shank.

In some embodiments, the third mounting part includes a third thread located on a side wall of the blade shank, and the fifth mounting part includes a fourth thread located on a side wall of the handle, the fourth thread being threadedly connected to the third thread.

In some embodiments, the fourth mounting part includes a fifth thread located on the side wall of the sleeve body, and the sixth mounting part includes a sixth thread located on a side wall of the handle, the sixth thread being threadedly connected to the fifth thread.

In some embodiments, the inner side wall of the sleeve body is provided with a first mounting groove, and the fourth mounting part includes an elastic ring, an outer ring of the elastic ring is fixedly connected to the first mounting groove, and a spacing between an inner ring and the outer ring of the elastic ring is greater than a depth of the first mounting groove; and an outer side wall of the end of the handle facing the blade shank is provided with a second mounting groove configured to accommodate the elastic ring.

In a second aspect, an embodiment of the present application provides a medical ultrasonic scalpel system, including a vibration source, and a medical ultrasonic scalpel according to any one of the embodiments described above, where the vibration source is connected to the medical ultrasonic scalpel and is configured to generate vibration.

In a third aspect, an embodiment of the present application provides a robot-assisted ultrasonic scalpel system, including a robot-assisted surgical device, and a medical ultrasonic scalpel system according to the embodiment described above, where the robot-assisted surgical device is connected to the medical ultrasonic scalpel of the medical ultrasonic scalpel system to control the movement of the medical ultrasonic scalpel.

The medical ultrasonic scalpel according to the embodiments of the present application can realize the connection and disconnection of the blade shank and the handle by means of the liquid flow sleeve, and can also realize the connection and disconnection of the liquid flow sleeve and the handle. During the assembly of the medical ultrasonic scalpel, no wrench is required, and the function of the wrench is realized by the liquid flow sleeve, thereby reducing the number of tools required for the assembly of the medical ultrasonic scalpel. Moreover, the structure of the liquid flow sleeve is complex, and the assembly is more convenient. Since repeated use of the wrench is not necessary, the assembly and disassembly process is faster, and the work efficiency is higher.

### BRIEF DESCRIPTION OF THE DRAWINGS

In the accompanying drawings, the same reference signs denote the same or similar components or elements throughout a plurality of accompanying drawings unless otherwise specified. These accompanying drawings are not necessarily drawn to scale. It should be understood that these accompanying drawings depict only some embodiments according to the present application herein and are not to be construed as limiting the scope of the present application.
Fig. 1 shows a schematic structural diagram of a medical ultrasonic scalpel according to an embodiment of the present application;
Fig. 2 shows a schematic cross-sectional view of the medical ultrasonic scalpel in Fig. 1;
Fig. 3 shows a schematic structural diagram of a liquid flow sleeve according to an embodiment of the present application;
Fig. 4 shows a schematic cross-sectional view of the liquid flow sleeve in Fig. 3;
Fig. 5 shows a schematic cross-sectional view of the liquid flow sleeve in Fig. 4 from another perspective;
Fig. 6 shows a front view of the liquid flow sleeve in Fig. 3;
Fig. 7 shows a top view of the liquid flow sleeve in Fig. 3;
Fig. 8 shows another schematic cross-sectional view of the liquid flow sleeve in Fig. 3;
Fig. 9 shows a schematic structural diagram of a blade shank in Fig. 1;
Fig. 10 shows a schematic structural diagram of a medical ultrasonic scalpel according to an embodiment of the present application;
Fig. 11 shows a schematic cross-sectional view of the medical ultrasonic scalpel in Fig. 10;
Fig. 12 shows a schematic structural diagram of the medical ultrasonic scalpel in another state according to an embodiment of the present application;
Fig. 13 shows a schematic cross-sectional view of the medical ultrasonic scalpel in Fig. 12;
Fig. 14 shows a schematic structural diagram of the medical ultrasonic scalpel in another state according to an embodiment of the present application;
Fig. 15 shows a schematic cross-sectional view of the medical ultrasonic scalpel in Fig. 14;
Fig. 16 shows another schematic cross-sectional view of the medical ultrasonic scalpel according to an embodiment of the present application;
Fig. 17 shows a schematic cross-sectional view of the medical ultrasonic scalpel in Fig. 16 in another state;
Fig. 18 shows a schematic cross-sectional view of the medical ultrasonic scalpel in Fig. 16 in another state;
Fig. 19 shows a schematic cross-sectional view of another liquid flow sleeve according to an embodiment of the present application;
Fig. 20 shows a schematic structural diagram of another handle according to an embodiment of the present application;
Fig. 21 shows a schematic structural diagram of another medical ultrasonic scalpel;
Fig. 22 shows a schematic cross-sectional view of the medical ultrasonic scalpel in Fig. 21;
Fig. 23 shows a schematic structural diagram of a medical ultrasonic scalpel in another state;
Fig. 24 shows a schematic cross-sectional view of the medical ultrasonic scalpel in Fig. 23;
Fig. 25 shows a schematic cross-sectional view of another liquid flow sleeve according to an embodiment of the present application;
Fig. 26 shows a schematic structural diagram of another blade shank according to an embodiment of the present application;
Fig. 27 shows a schematic structural diagram of another medical ultrasonic scalpel according to an embodiment of the present application;
Fig. 28 shows a schematic structural diagram of another medical ultrasonic scalpel;
Fig. 29 shows a schematic cross-sectional view of the medical ultrasonic scalpel in Fig. 28;
Fig. 30 shows a schematic structural diagram of a medical ultrasonic scalpel in another state; and
Fig. 31 shows a schematic cross-sectional view of the medical ultrasonic scalpel in Fig. 30.

List of reference signs:
100. liquid flow sleeve; 101. sleeve body; 111. mounting cavity; 112. first mounting hole; 113. fourth mounting part; 114. first mounting groove; 115. elastic ring; 116. first mounting part; 117. second mounting hole; 118. elastic piece; 119. limiting protrusion; 102. first protruding part; 121. first portion; 122. second portion; 1221. first surface; 1222. second surface; 103. protrusion; 200. blade shank; 201. third mounting part; 202. second protruding part; 221. third surface; 222. fourth surface; 203. second mounting part; 204. boss; 205. blade tip; 300. handle; 301. fifth mounting part; 302. sixth mounting part; 303. second mounting groove.

### DETAILED DESCRIPTION OF EMBODIMENTS

Only some exemplary embodiments will be briefly described below. As can be appreciated by those skilled in the art, the described embodiments can be modified in various ways without departing from the spirit or scope of the present application. Accordingly, the accompanying drawings and the description are considered illustrative in nature rather than limited.

At present, from the perspective of market development, medical ultrasonic scalpels have also been gradually applied to surgical operations. Due to the characteristics of ultrasound, when the medical ultrasonic scalpel comes into contact with bone tissue with relatively high hardness, the bone tissue is not easily deformed, thereby enabling cutting or crushing at the contact position. In contrast, when the medical ultrasonic scalpel comes into contact with soft tissue, the soft tissue, under the action of elasticity, will be displaced or deformed, and will perform micro-vibration along with the vibration of the medical ultrasonic scalpel, thereby offsetting the energy at the blade shank and avoiding cutting or crushing. Therefore, especially for surgical operations where the surgical site is located near the interface between the bone and the soft tissue, the medical ultrasonic scalpel has significant advantages in bone cutting. During surgery, a medical ultrasonic scalpel will generate friction with body tissues, thereby generating heat. In most cases, the heat generated by friction will damage cellular tissues, cause postoperative complications, and produce adverse effects. Therefore, when using the medical ultrasonic scalpel for surgery, a liquid flow system is typically provided to cool friction hotspots on a blade shank by means of a liquid flow sleeve.

In the related art, a medical ultrasonic scalpel includes a handle, a blade shank and a liquid flow sleeve. The blade shank is connected to the handle, and the liquid flow sleeve is sleeved on the blade shank and connected to the handle. During the assembly of the medical ultrasonic scalpel, a wrench is first used to connect the blade shank to the handle, and the wrench is then used to connect the liquid flow sleeve to the handle. The use of the wrench increases the number of tools in the assembly process, and the complicated structure of the wrench also makes the assembly process inconvenient.

In order to improve the convenience of installing the medical ultrasonic scalpel, an embodiment of the present application provides a medical ultrasonic scalpel, in which a liquid flow sleeve can realize the connection between the liquid flow sleeve and a handle, and can also realize the connection between a blade shank and the liquid flow sleeve, without the need for a wrench for assembly, thereby making the assembly process of the medical ultrasonic scalpel more convenient.

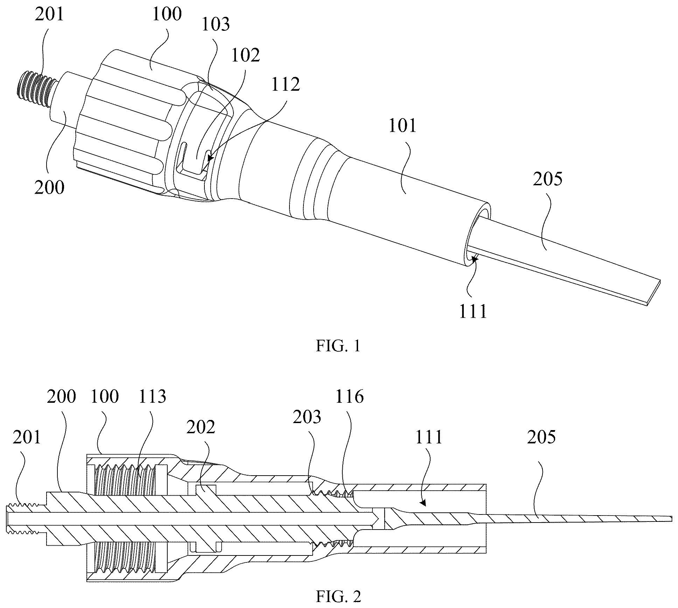

An embodiment of the present application provides a medical ultrasonic scalpel. Fig. 1 shows a schematic structural diagram of the medical ultrasonic scalpel according to the embodiment of the present application. Fig. 2 shows a schematic cross-sectional view of the medical ultrasonic scalpel in Fig. 1. Referring to Figs. 1 and 2, the medical ultrasonic scalpel includes a liquid flow sleeve 100 and a blade shank 200. An extension direction X of the blade shank 200 is the same as an extension direction of the liquid flow sleeve 100.

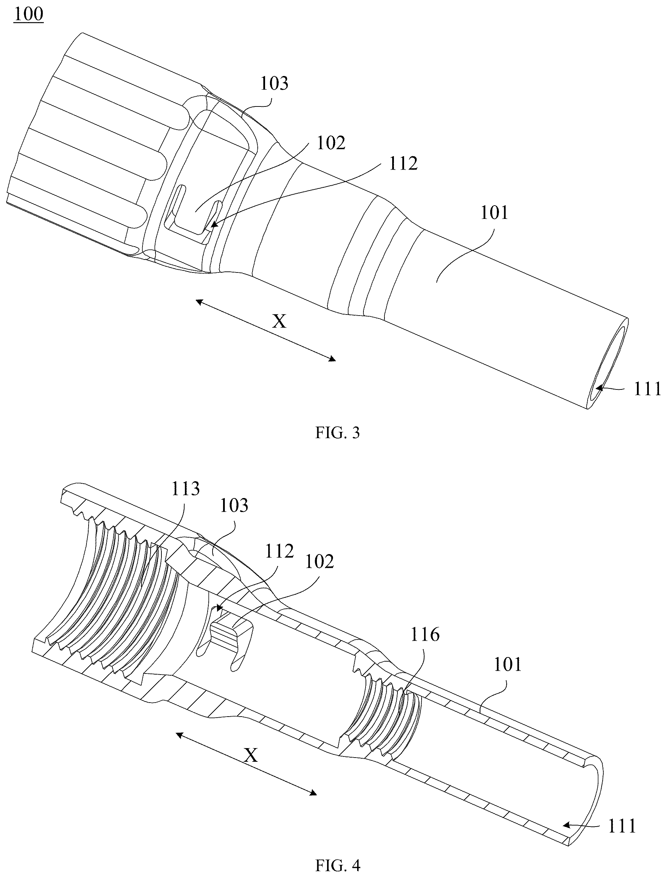

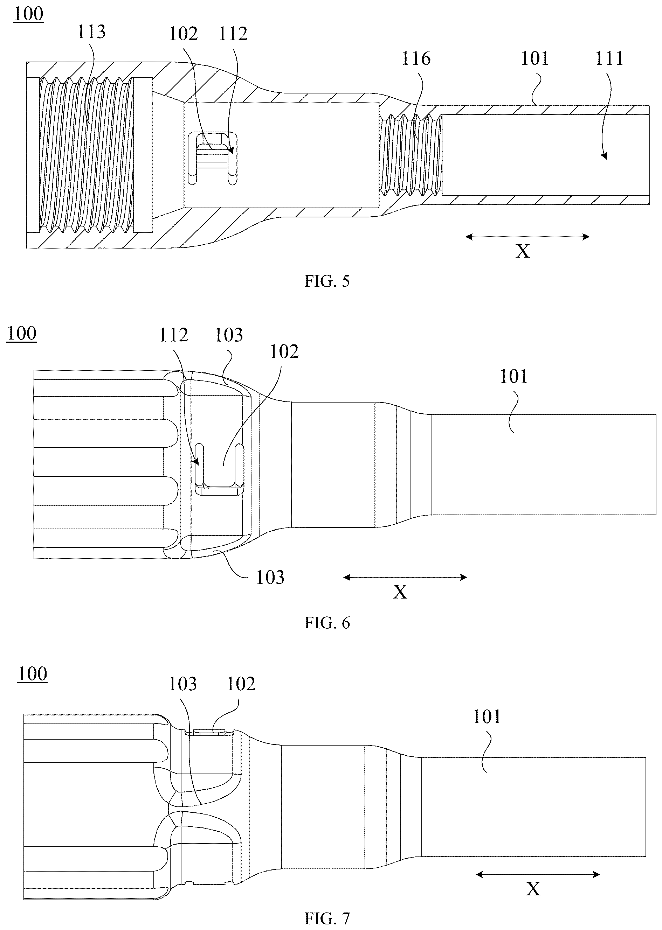

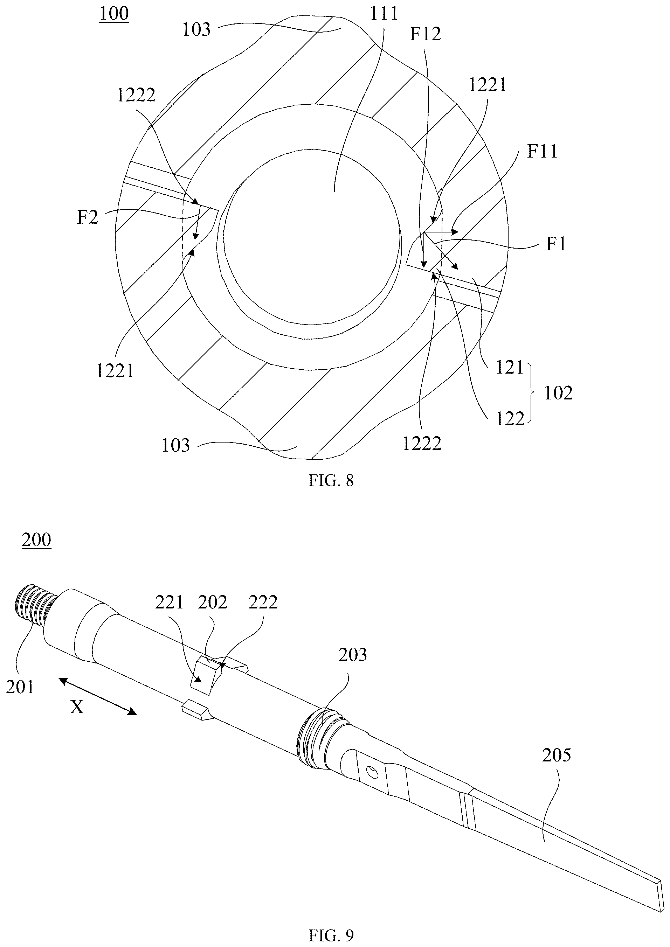

Fig. 3 shows a schematic structural diagram of a liquid flow sleeve according to an embodiment of the present application. Fig. 4 shows a schematic cross-sectional view of the liquid flow sleeve in Fig. 3. Fig. 5 shows a schematic cross-sectional view of the liquid flow sleeve in Fig. 4 from another perspective. Fig. 6 shows a front view of the liquid flow sleeve in Fig. 3. Fig. 7 shows a top view of the liquid flow sleeve in Fig. 3. Fig. 8 shows another schematic cross-sectional view of the liquid flow sleeve in Fig. 3. Referring to Figs. 3 to 8, the liquid flow sleeve 100 includes a sleeve body 101 and a first protruding part 102. The sleeve body 101 is provided with a mounting cavity 111 running through two opposite ends of the sleeve body 101. The first protruding part 102 is located on an inner side wall of the sleeve body 101.

Referring to Figs. 1 and 2, the blade shank 200 runs through the mounting cavity 111. The blade shank 200 is pivotally connected to the liquid flow sleeve 100, and the blade shank 200 and the liquid flow sleeve 100 are movable in the extension direction X of the liquid flow sleeve 100. Fig. 9 shows a schematic structural diagram of the blade shank in Fig. 1. Referring to Fig. 9, an outer side wall of the blade shank 200 is provided with a second protruding part 202 corresponding to the first protruding part 102.

The first protruding part 102 and the second protruding part 202 are configured to cooperate such that when the liquid flow sleeve 100 rotates in a first rotation direction A, the first protruding part 102 and the second protruding part 202 are offset from each other, so as to enable the liquid flow sleeve 100 to move in the extension direction X, and when the liquid flow sleeve 100 rotates in a second rotation direction B, the first protruding part 102 abuts against the second protruding part 202, so as to drive the blade shank 200 to rotate in the second rotation direction B. The first rotation direction A is opposite to the second rotation direction B.

When the medical ultrasonic scalpel according to the embodiments of the present application is in use, after the liquid flow sleeve 100 is sleeved on the blade shank 200, the liquid flow sleeve 100 is rotated. Assuming that the rotation direction at this point is the first rotation direction A, the second protruding part 202 and the first protruding part 102 are offset from each other, so as to enable the liquid flow sleeve 100 to move in the extension direction X, so that the liquid flow sleeve 100 can be connected to the handle. When the liquid flow sleeve 100 is disconnected from the blade shank 200, the liquid flow sleeve 100 is first disconnected from the handle, and the liquid flow sleeve 100 is then rotated, assuming that the rotation direction at this point is the second rotation direction B, the first rotation direction A being opposite to the second rotation direction B. For example, the first rotation direction A may be counterclockwise, and the second rotation direction B may be clockwise. At this point, the second protruding part 202 abuts against the first protruding part 102. The second protruding part 202 applies a thrust to the first protruding part 102, causing the blade shank 200 to rotate, thereby disconnecting the blade shank 200 from the handle.

The medical ultrasonic scalpel according to the embodiments of the present application can realize the connection and disconnection of the blade shank 200 and the handle, and can also realize the connection and disconnection of the liquid flow sleeve 100 and the handle. During the assembly of the medical ultrasonic scalpel, no wrench is required, and the function of the wrench is realized by the liquid flow sleeve 100, thereby reducing the number of tools required for the assembly of the medical ultrasonic scalpel. Moreover, the structure of the liquid flow sleeve 100 is simple, and the assembly is more convenient. Since repeated use of the wrench is not necessary, the assembly and disassembly process is faster, and the work efficiency is higher.

Referring to Figs. 1 to 6, the sleeve body 101 is provided with at least one first mounting hole 112 running through a side wall of the sleeve body 101; and the first protruding part 102 comprises a first portion 121 and a second portion 122 connected to each other. The first portion 121 is located in the first mounting hole 112, and the first portion 121 is connected to the sleeve body 101. The second portion 122 is located in the mounting cavity 111, and the second portion 122 and the second protruding part 202 are configured such that, when the liquid flow sleeve 100 rotates in the first rotation direction A, the second protruding part 202 pushes the second portion 122 to move into the first mounting hole 112, and when the liquid flow sleeve 100 rotates in the second rotation direction B, the second portion 122 abuts against the second protruding part 202.

In an embodiment of the present application, after the liquid flow sleeve 100 is sleeved on the blade shank 200, the liquid flow sleeve 100 is rotated in the first rotation direction A, the second protruding part 202 abuts against the second portion 122, and the second protruding part 202 applies a thrust to the second portion 122. When the thrust is less than or equal to a certain thrust value (e.g., a first preset thrust value), the liquid flow sleeve 100 drives the blade shank 200 to rotate, and the rotation of the blade shank 200 causes the blade shank 200 to be threadedly connected to the handle. When the blade shank 200 is already threadedly connected to the handle and the liquid flow sleeve 100 continues to be rotated, in which case the blade shank 200 has been connected to the handle, the blade shank 200 does not rotate with the liquid flow sleeve 100, so that the acting force between the second protruding part 202 and the second portion 122 increases. When the thrust applied by the second protruding part 202 to the second portion 122 is greater than a certain thrust value, the second portion 122 moves into the first mounting hole 112, so that the second protruding part can no longer contact the first protruding part 102, and does not apply thrust to the blade shank 200, and the blade shank 200 does not rotate, while the liquid flow sleeve 100 can continue to be rotated and is connected to the handle. When the liquid flow sleeve 100 is disconnected from the blade shank 200, the liquid flow sleeve 100 is first disconnected from the handle, and the liquid flow sleeve 100 is then rotated, assuming that the rotation direction at this point is the second rotation direction B, the first rotation direction A being opposite to the second rotation direction B. For example, the first rotation direction A may be counterclockwise, and the second rotation direction B may be clockwise. When the second protruding part 202 abuts against the second portion 122 of the first protruding part 102, the second protruding part applies a thrust to the second portion 122, causing the blade shank 200 to rotate, thereby disconnecting the blade shank 200 from the handle.

Referring to Fig. 8, the second portion 122 includes a first surface 1221 and a second surface 1222. The first surface 1221 and the second surface 1222 are both parallel to the extension direction X of the liquid flow sleeve 100. The first surface 1221 is configured such that, when a thrust applied to the first surface 1221 toward the second surface 1222 is greater than a first preset thrust value, the second portion 122 is moved into the first mounting hole 112, and the second surface 1222 is configured such that, when a thrust applied to the second surface 1222 toward the first surface 1221 is greater than a second preset thrust value, the liquid flow sleeve 100 is moved.

In an embodiment of the present application, the sleeve body 101 is of a tubular structure. By way of example, the sleeve body 101 may be in the shape of a cylindrical tube. For example, the sleeve body 101 may be a cylindrical tube with the same diameter at all portions, or the sleeve body 101 may be formed by combining cylindrical tubes of different diameters.

In an embodiment of the present application, the blade shank 200 passes through the mounting cavity 111 of the sleeve body 101, such that the liquid flow sleeve 100 is sleeved on the blade shank 200.

In an embodiment of the present application, the first mounting hole 112 runs through the side wall of the sleeve body 101 and is in communication with the mounting cavity 111. The shape of the first mounting hole 112 may be set according to requirements, for example, the first mounting hole 112 may be rectangular.

In an embodiment of the present application, a side edge of the first protruding part 102 is connected to the first mounting hole 112, that is, connected to the sleeve body 101, and other side edges of the first protruding part 102 are free, such that the first protruding part 102 can rotate around the side edge connected to the sleeve body 101.

In an embodiment of the present application, it is possible that the first protruding part 102 and the sleeve body 101 are formed integrally, or that the first mounting hole 112 is formed in the sleeve body 101, and the first protruding part 102 is then connected to the sleeve body 101, for example, the first protruding part 102 is welded to the sleeve body 101.

In an embodiment of the present application, the shape of the first protruding part 102 may be determined according to the shape of the first mounting hole 112. For example, the first portion 121 of the first protruding part 102 may be a cuboid, and the second portion 122 may be a protrusion of the first portion 121 and located in the mounting cavity 111. By way of example, the second portion 122 may be a prism having a height direction parallel to the extension direction X of the liquid flow sleeve 100. One of side surfaces of the prism is connected to the first portion 121, and two of the side surfaces of the prism serve as the first surface 1221 and the second surface 1222, respectively.

In an embodiment of the present application, the first portion 121 and the second portion 122 are integrated to jointly form the first protruding part 102. In the figures of the present application, in order to clearly show the first portion 121 and the second portion 122, a dashed line is added between the first portion 121 and the second portion 122 for distinction.

According to some embodiments of the present application, a central axis of rotation of the liquid flow sleeve 100 is located in a plane where the second surface 1222 is located, and the central axis of rotation of the liquid flow sleeve 100 is offset from each other from a plane where the first surface 1221 is located.

In an embodiment of the present application, the plane where the second surface 1222 is located passes through the center of the liquid flow sleeve 100, and the plane where the first surface 1221 is located does not pass through the center of the liquid flow sleeve 100. In this way, a thrust F1 is applied to the first surface 1221 toward the second surface 1222 in the mounting cavity 111. The thrust F1 is perpendicular to the first surface 1221, and the thrust F1 can be decomposed into a first component F11 and a second component F12. An extension line of the first component F11 passes through the center of the liquid flow sleeve 100, that is, the first component F11 is parallel to the diameter of the liquid flow sleeve 100 at this position, and the second component F12 is perpendicular to the first component F11. Since the first component F11 points toward the first mounting hole 112, which is equivalent to applying a force to the second portion 122 toward the first mounting hole 112. When reaching a certain set force, the first component F11 will push the second portion 122 to move toward the first mounting hole 112. After the second portion 122 is moved into the first mounting hole 112, no further contact can be made with the second portion 122, that is, no further thrust can be applied to the first surface 1221.

In an embodiment of the present application, a thrust F2 is applied to the second surface 1222 toward the first surface 1221 in the mounting cavity 111. The thrust F2 is perpendicular to the second surface 1222. Since the plane where the second surface 1222 is located passes through the center of the liquid flow sleeve 100, that is, the thrust F2 has no component pointing toward the outside of the liquid flow sleeve 100, the second portion 122 will not move toward the first mounting hole 112 under the action of the thrust F2. When the thrust F2 is greater than the second preset thrust value, the second portion 122 is pushed to move in the direction of the thrust F2, thereby driving the liquid flow sleeve 100 to move.

It should be noted that since the liquid flow sleeve 100 in the present application is a cylindrical tube, the liquid flow sleeve 100 can be rotated under the action of the thrust F2.

When the liquid flow sleeve 100 according to the embodiment of the present application is in use, the blade shank 200 is provided with the second protruding part 202 matching the first protruding part 102, and after the liquid flow sleeve 100 is sleeved on the blade shank 200, the liquid flow sleeve 100 is rotated. Assuming that the rotation direction at this point is the first rotation direction A, the second protruding part 202 abuts against the first surface 1221 of the first protruding part 102, and the second protruding part 202 applies a thrust to the first surface 1221 toward the second surface 1222. When the thrust is less than or equal to the first preset thrust value, the liquid flow sleeve 100 drives the blade shank 200 to rotate, and the rotation of the blade shank 200 causes the blade shank 200 to be threadedly connected to the handle. When the blade shank 200 is already threadedly connected to the handle and the liquid flow sleeve 100 continues to be rotated, in which case the blade shank 200 has been connected to the handle, the blade shank 200 does not rotate with the liquid flow sleeve 100, so that the acting force between the second protruding part 202 and the first surface 1221 increases. When the thrust applied by the second protruding part 202 to the first surface 1221 is greater than the first preset thrust value, the second portion 122 moves into the first mounting hole 112, so that the second protruding part 202 can no longer contact the first protruding part 102, and does not apply thrust to the blade shank 200, and the blade shank 200 does not rotate, while the liquid flow sleeve 100 can continue to be rotated and is connected to the handle. When the liquid flow sleeve 100 is disconnected from the blade shank 200, the liquid flow sleeve 100 is first disconnected from the handle, and the liquid flow sleeve 100 is then rotated, assuming that the rotation direction at this point is the second rotation direction B, the first rotation direction A being opposite to the second rotation direction B. For example, the first rotation direction A may be counterclockwise, and the second rotation direction B may be clockwise. When the second protruding part 202 abuts against the second surface 1222 of the first protruding part 102, the second protruding part 202 applies a thrust to the second surface 1222 toward the first surface 1221, driving the blade shank 200 to rotate, thereby disconnecting the blade shank 200 from the handle.

The medical ultrasonic scalpel according to the embodiment of the present application can realize the connection and disconnection of the blade shank 200 and the handle by means of the liquid flow sleeve 100, and can also realize the connection and disconnection of the liquid flow sleeve 100 and the handle. During the assembly of the medical ultrasonic scalpel, no wrench is required, and the function of the wrench is realized by the liquid flow sleeve 100, thereby achieving higher working efficiency.

In an embodiment of the present application, the sleeve body 101 is provided with two first mounting holes 112. The two first mounting holes 112 are symmetrically spaced apart in a circumferential direction of the side wall of the sleeve body 101. The liquid flow sleeve 100 includes two first protruding parts 102 corresponding to the two first mounting holes 112.

In the embodiment of the present application, the two first mounting holes 112 and the two first protruding parts 102 are provided, so that even if one of the two first mounting holes or the two first protruding parts is damaged, the function of the liquid flow sleeve 100 can still be retained. Moreover, when the liquid flow sleeve 100 is rotated, it is only necessary to rotate at most half a turn to make the second protruding part abut against the first protruding part 102, thereby saving effort.

Referring to Figs. 3, 4 and 6 to 8, the liquid flow sleeve 100 further includes a protrusion 103. The protrusion 103 is connected to an outer side wall of the sleeve body 101, and the protrusion 103 is arranged spaced apart from the first mounting hole 112.

In the embodiment of the present application, it is more convenient to rotate the liquid flow sleeve 100 by holding the protrusion 103.

By way of example, the liquid flow sleeve 100 may include two protrusions 103. The two first mounting holes 112 and the two protrusions 103 may be evenly spaced apart in the circumferential direction of the liquid flow sleeve 100.

In some embodiments of the present application, the liquid flow sleeve 100 may be made of rigid metal or plastic.

In some other embodiments of the present application, the portion of the liquid flow sleeve 100 that is connected to the blade shank may be made of rigid metal or plastic, and the end of the liquid flow sleeve 100 away from a fourth mounting part 113 may be made of a soft material such as silicone, so that the portion of the liquid flow sleeve 100 that is likely to contact bone tissue has better elasticity, thereby reducing damage to the bone tissue.

According to some embodiments of the present application, referring to Fig. 9, the second protruding part 202 includes a third surface 221 and a fourth surface 222. The third surface 221 and the fourth surface 222 are both parallel to the extension direction X of the liquid flow sleeve 100. The third surface 221 is configured to apply a thrust to the first surface 1221 toward the second surface 1222, and the fourth surface 222 is configured to apply a thrust to the second surface 1222 toward the first surface 1221. The second protruding part 202 matches the shape of the first protruding part 102, so that when the blade shank 200 rotates in either of two different directions, the second protruding part 202 has a portion capable of contacting the first protruding part 102.

In an embodiment of the present application, the blade shank 200 is provided with a blade tip 205. The blade tip 205 extends beyond the blade shank 200 for cutting bone tissue.

In an embodiment of the present application, the blade shank 200 is pivotally connected to the liquid flow sleeve 100, allowing mutual rotation between the blade shank 200 and the liquid flow sleeve 100, with the central axis of rotation being the same as the extension direction X of the liquid flow sleeve 100.

In an embodiment of the present application, the second protruding part 202 may be integrally formed with a main body of the blade shank 200, or the second protruding part 202 may be welded to the main body of the blade shank 200.

In an embodiment of the present application, the second protruding part 202 may be a prism having a height direction parallel to the extension direction X of the liquid flow sleeve 100. One of side surfaces of the prism is connected to the main body of the blade shank 200, and two of the side surfaces of the prism serve as the third surface 221 and the fourth surface 222, respectively.

In an embodiment of the present application, during rotation of the liquid flow sleeve 100 in the first rotation direction A, the third surface 221 abuts against the first surface 1221, and during rotation of the liquid flow sleeve 100 in the second rotation direction B, the fourth surface 222 abuts against the second surface 1222.

In an embodiment of the present application, the number of second protruding parts 202 is greater than or equal to 1 and less than or equal to 4.

By way of example, the outer side wall of the blade shank 200 may be provided with four second protruding parts 202. The four second protruding parts 202 are symmetrically spaced apart in the circumference direction of the blade shank 200. Two second protruding parts 202 are provided between two adjacent first protruding parts 102, so that when the liquid flow sleeve 100 is rotated, it is only necessary to rotate at most a quarter turn to make the second protruding part abut against the first protruding part 102, thereby saving effort.

In an embodiment of the present application, during the assembly of the medical ultrasonic scalpel and the handle, it is possible to cooperate the first protruding part 102 with the second protruding part 202 to allow assembly and disassembly of the medical ultrasonic scalpel and the handle without using a wrench, so that the assembly is more convenient, and fewer tools are required during the assembly process.

According to some embodiments of the present application, one end of the blade shank 200 is provided with a third mounting part 201, and one end of the sleeve body 101 is provided with a fourth mounting part 113.

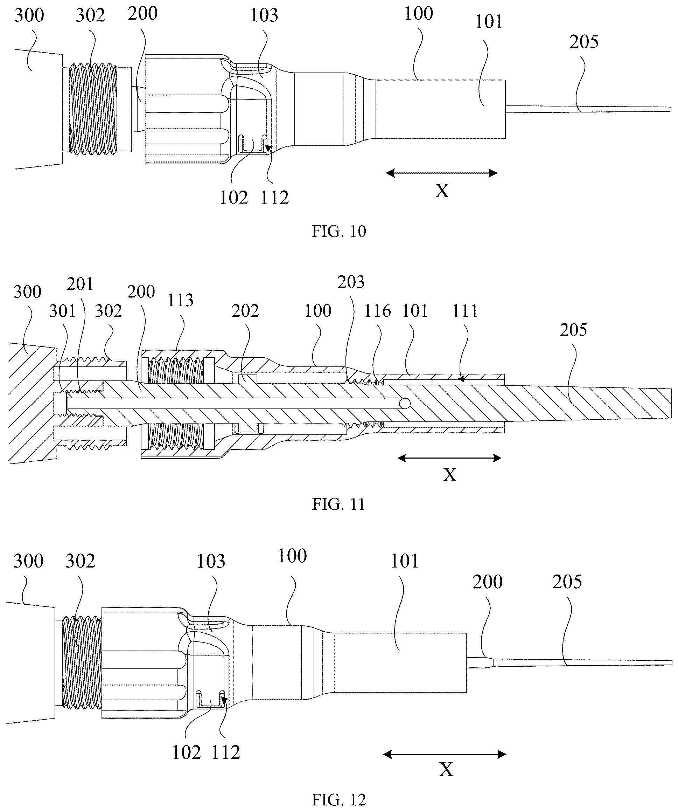

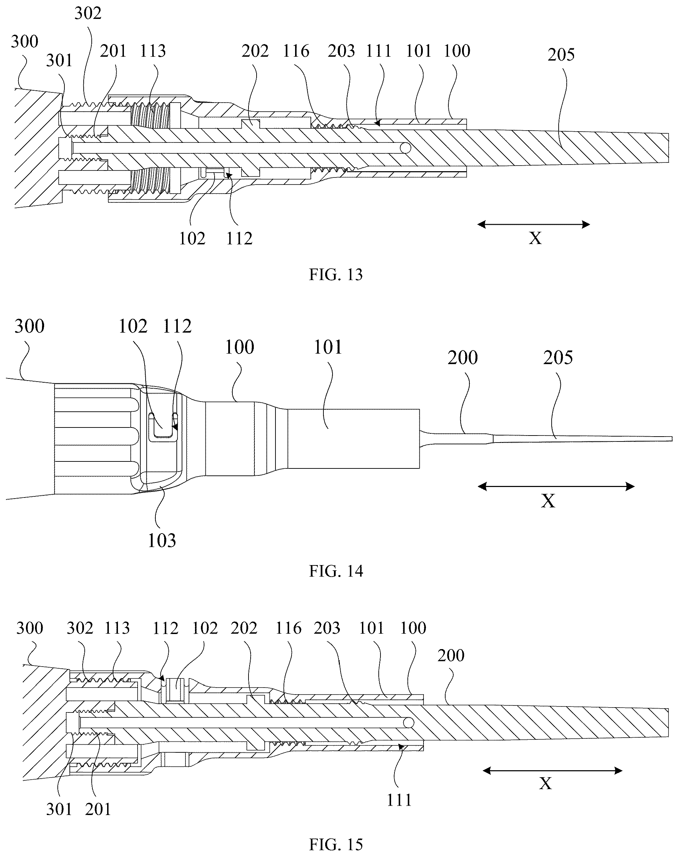

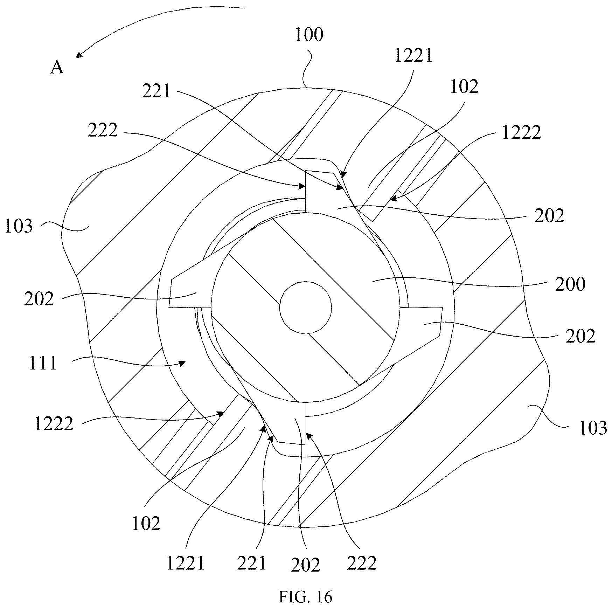

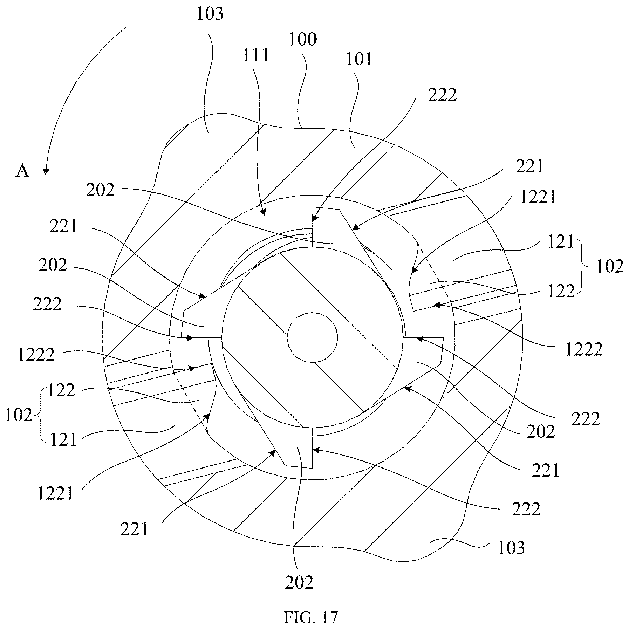

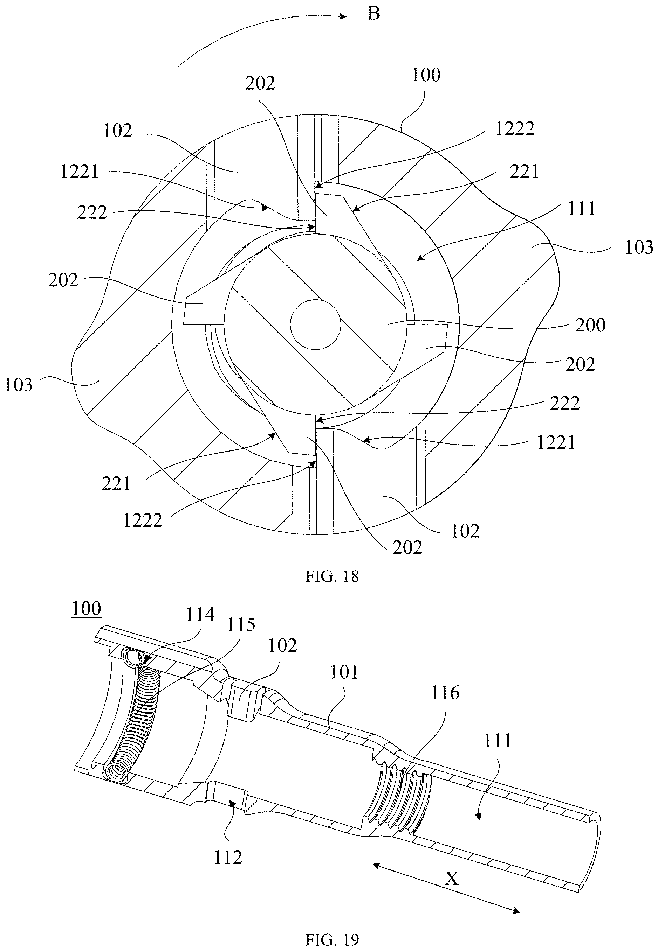

Fig. 10 shows a schematic structural diagram of a medical ultrasonic scalpel according to an embodiment of the present application. Fig. 11 shows a schematic cross-sectional view of the medical ultrasonic scalpel in Fig. 10. Fig. 12 shows a schematic structural diagram of the medical ultrasonic scalpel in another state according to an embodiment of the present application. Fig. 13 shows a schematic cross-sectional view of the medical ultrasonic scalpel in Fig. 12. Fig. 14 shows a schematic structural diagram of the medical ultrasonic scalpel in another state according to an embodiment of the present application. Fig. 15 shows a schematic cross-sectional view of the medical ultrasonic scalpel in Fig. 14. Fig. 16 shows another schematic cross-sectional view of the medical ultrasonic scalpel according to an embodiment of the present application. Fig. 17 shows a schematic cross-sectional view of the medical ultrasonic scalpel in Fig. 16 in another state. Fig. 18 shows a schematic cross-sectional view of the medical ultrasonic scalpel in Fig. 16 in another state. Referring to Figs. 10 to 18, the medical ultrasonic scalpel further includes the handle 300. The handle 300 is provided with a fifth mounting part 301 connected to the third mounting part 201, and a sixth mounting part 302 connected to the fourth mounting part 113. The fifth mounting part 301 and the sixth mounting part 302 are both located at an end of the handle 300 facing the blade shank 200.

In an embodiment of the present application, the third mounting part 201 is used for connection with the fifth mounting part 301 of the handle 300, so that the blade shank 200 is connected to the handle 300.

In an embodiment of the present application, in the extension direction X of the liquid flow sleeve 100, the first mounting hole 112 and the fourth mounting part 113 are spaced apart from each other.

In an embodiment of the present application, the fourth mounting part 113 is used for connection with the sixth mounting part 302 of the handle 300, so that the liquid flow sleeve 100 is connected to the handle 300.

In an embodiment of the present application, the third mounting part 201 includes a third thread located on a side wall of the blade shank 200, and the fifth mounting part 301 includes a fourth thread located on a side wall of the handle 300. The fourth thread is threadedly connected to the third thread.

In an embodiment of the present application, the third mounting part 201 is threadedly connected to the fifth mounting part 301, thereby realizing the connection between the blade shank 200 and the handle 300. For example, the third mounting part 201 may be an external thread, and correspondingly, the fifth mounting part 301 may be an internal thread.

The assembly and disassembly of the medical ultrasonic scalpel are described below with reference with Figs. 10 to 18.

In an initial state, the first protruding part 102 and the second protruding part 202 may be in a contact state or may be in a non-contact state.

In step S10, the handle 300 and the blade shank 200 are assembled.

In one implementation of the present application, the blade shank 200 may be directly rotated such that the third mounting part 201 is threadedly connected to the fifth mounting part 301, thereby realizing the connection between the blade shank 200 and the handle 300, as shown in Figs. 10 and 11.

In another implementation of the present application, the liquid flow sleeve 100 may be rotated in the first rotation direction A first, as shown in Fig. 16, such that the first protruding part 102 comes into contact with the second protruding part 202, and the third surface 221 abuts against the first surface 1221. In this case, the blade shank 200 is not connected to the handle 300 and does not block the rotation of the blade shank 200. Driven by the liquid flow sleeve 100, the blade shank 200 rotates in the first rotation direction A, such that the third mounting part 201 is threadedly connected to the fifth mounting part 301, thereby realizing the connection between the blade shank 200 and the handle 300, as shown in Figs. 10 and 11. Moreover, the fourth mounting part 113 and the sixth mounting part 302 gradually approach each other during this process.

In step S20, the handle 300 and the liquid flow sleeve 100 are assembled.

The liquid flow sleeve 100 continues to be rotated in the first rotation direction A. Since the blade shank 200 is connected to the handle 300, the blade shank 200 cannot rotate, so that the acting force between the first protruding part 102 and the second protruding part 202 increases. When the acting force between the first protruding part 102 and the second protruding part 202 is greater than the first preset thrust value, the second portion 122 moves into the first mounting hole 112, and the first protruding part 102 cannot be in contact with the second protruding part 202, thereby not blocking the rotation of the liquid flow sleeve 100, as shown in Fig. 17. The blade shank 200 and the liquid flow sleeve 100 move relative to each other in the extension direction of the liquid flow sleeve 100, such that the fourth mounting part 113 coincides with the sixth mounting part 302, and the handle 300 and the liquid flow sleeve 100 are gradually connected to each other, as shown in Figs. 12 and 13, until the handle 300 and the liquid flow sleeve 100 are fully connected to each other, as shown in Figs. 14 and 15.

Compared with Fig. 16, in Fig. 17, one first protruding part 102 passes over at least two second protruding parts 202 during rotation.

In step S30, the handle 300 and the liquid flow sleeve 100 are disassembled.

The liquid flow sleeve 100 is rotated in the second rotation direction B, such that the handle 300 gradually moves away from the liquid flow sleeve 100. The medical ultrasonic scalpel changes from the state shown in Figs. 14 and 15 to the state shown in Figs. 12 and 13 until the handle 300 and the liquid flow sleeve 100 are completely disassembled, reaching the state shown in Figs. 10 and 11.

In step S40, the handle 300 and the blade shank 200 are disassembled.

The liquid flow sleeve 100 continues to be rotated in the second rotation direction B, such that the first protruding part 102 comes into contact with the second protruding part 202, and the fourth surface 222 abuts against the second surface 1222, as shown in Fig. 18. When the thrust applied by the fourth surface 222 to the second surface 1222 is greater than the second preset thrust value, the liquid flow sleeve 100 drives the blade shank 200 to rotate, thereby disassembling the handle 300 and the blade shank 200.

In an embodiment of the present application, a chip with an identifiable program may be embedded in one end of the liquid flow sleeve 100 facing the handle 300. After the liquid flow sleeve 100 is connected to the handle 300, information such as models of the liquid flow sleeve 100 and the blade shank 200 can be identified.

The above assembly and disconnection processes are described by means of the threaded connection between the handle 300 and the liquid flow sleeve 100.

By way of example, referring to Figs. 4 and 5, the fourth mounting part 113 is a fifth thread on the side wall of the sleeve body 101. Referring to Figs. 10, 11, 13, 14, 16 and 17, when the fourth mounting part 113 is the fifth thread on the side wall of the sleeve body 101, the sixth mounting part 302 is a sixth thread on the side wall of the handle 300 that is threadedly connected to the fifth thread. By means of the threads, the connection between the handle 300 and the liquid flow sleeve 100 is more convenient, and the assembly and disassembly process is easier.

By way of example, the fifth thread may be an internal thread, and the sixth thread may be an external thread.

In an embodiment of the present application, the fifth mounting part 301 and the sixth mounting part 302 are both located at the end of the handle 300 facing the medical ultrasonic scalpel, facilitating the connection of the handle 300 with the blade shank 200 and the liquid flow sleeve 100.

In other implementations, the handle 300 and the liquid flow sleeve 100 may be connected in other manners.

Fig. 19 shows a schematic cross-sectional view of another liquid flow sleeve according to an embodiment of the present application. Referring to Fig. 19, the inner side wall of the sleeve body 101 is provided with a first mounting groove 114, and the fourth mounting part 113 (not shown in Fig. 19) includes an elastic ring 115. An outer ring of the elastic ring 115 is fixedly connected to the first mounting groove 114, and a spacing between an inner ring and the outer ring of the elastic ring 115 is greater than a depth of the first mounting groove 114.

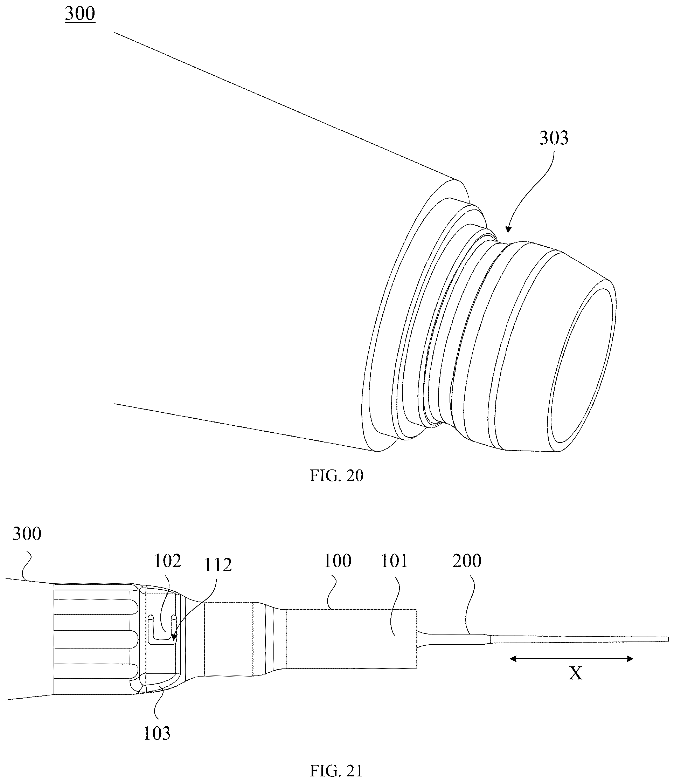

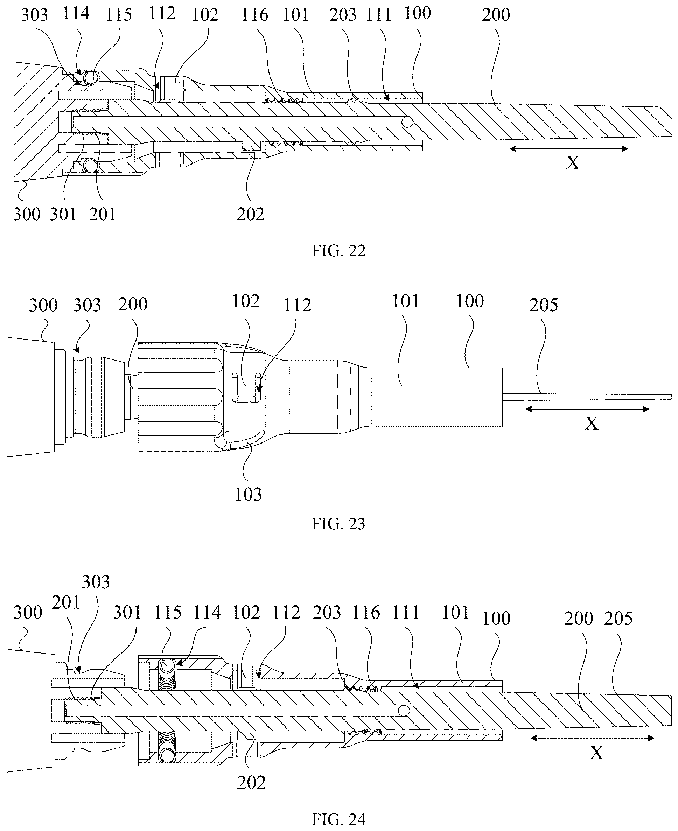

Fig. 20 shows a schematic structural diagram of another handle according to an embodiment of the present application. Fig. 21 shows a schematic structural diagram of another medical ultrasonic scalpel. Fig. 22 shows a schematic cross-sectional view of the medical ultrasonic scalpel in Fig. 21. Fig. 23 shows a schematic structural diagram of the medical ultrasonic scalpel in another state. Fig. 24 shows a schematic cross-sectional view of the medical ultrasonic scalpel in Fig. 23. When the fourth mounting part 113 includes the elastic ring 115, the outer side wall of the end of the handle 300 facing the medical ultrasonic scalpel is provided with a second mounting groove 303. The second mounting groove 303 is configured to accommodate the elastic ring 115.

In an embodiment of the present application, before assembly of the above structure, a portion of the elastic ring 115 is located inside the first mounting groove 114. Since the spacing between the inner ring and the outer ring of the elastic ring 115 is greater than the depth of the first mounting groove 114, another portion of the elastic ring 115 protrudes from the first mounting groove 114. When the handle 300 is connected to the blade shank 200, the liquid flow sleeve 100 is pushed toward one side of the handle 300 in the extension direction X of the liquid flow sleeve 100, so that the other portion of the elastic ring 115 is embedded into the second mounting groove 303, that is, the elastic ring 115 is encased by the first mounting groove 114 and the second mounting groove 303. Moreover, the elastic ring 115 blocks the movement between the handle 300 and the liquid flow sleeve 100, thereby connecting the handle 300 to the liquid flow sleeve 100.

In the embodiment of the present application, since the elastic ring 115 is elastic and deformable, when the handle 300 and the blade shank 200 are disassembled, the liquid flow sleeve 100 is pushed toward the side away from the handle 300 in the extension direction X of the liquid flow sleeve 100. When a force applied to push the liquid flow sleeve 100 is sufficiently large, the elastic ring 115 is deformed, and the elastic ring 115 is disengaged from the second mounting groove 303, thereby realizing disassembly of the handle 300 and the liquid flow sleeve 100.

It should be noted that, during a surgical operation using the medical ultrasonic scalpel, a force applied to the liquid flow sleeve 100 is very small and will not cause the handle 300 and the liquid flow sleeve 100 to be separated from each other.

In an embodiment of the present application, the elastic ring 115 may be a rubber ring, a silicone ring, or a spring ring.

Referring to Figs. 4 and 5, the sleeve body 101 is also provided with a first mounting part 116. In the extension direction X of the liquid flow sleeve 100, the first mounting hole 112 is located between the fourth mounting part 113 and the first mounting part 116.

Referring to Fig. 2, when the sleeve body 101 is provided with the first mounting part 116, the end of the blade shank 200 close to the first mounting part 116 is provided with the second mounting part 203 connected to the first mounting part 116.

In an embodiment of the present application, connecting the liquid flow sleeve 100 to the blade shank 200 via the first mounting part 116 and the second mounting part 203 can improve the stability of the liquid flow sleeve 100.

In some embodiments of the present application, referring to Figs. 4 and 5, the first mounting part 116 is a first thread on the side wall of the sleeve body 101. Referring to Fig. 2, when the first mounting part 116 is the first thread on the side wall of the sleeve body 101, the second mounting part 203 is a second thread on the side wall of the blade shank 200 that is threadedly connected to the first thread.

In the embodiments of the present application, during assembly and disassembly of the medical ultrasonic scalpel, the assembly and disassembly of the liquid flow sleeve 100 and the blade shank 200 can be achieved by rotating the liquid flow sleeve 100. Connecting the liquid flow sleeve 100 to the blade shank 200 via threads is more convenient, and the assembly and disassembly process is simpler.

By way of example, the first thread may be an internal thread, and the second thread may be an external thread.

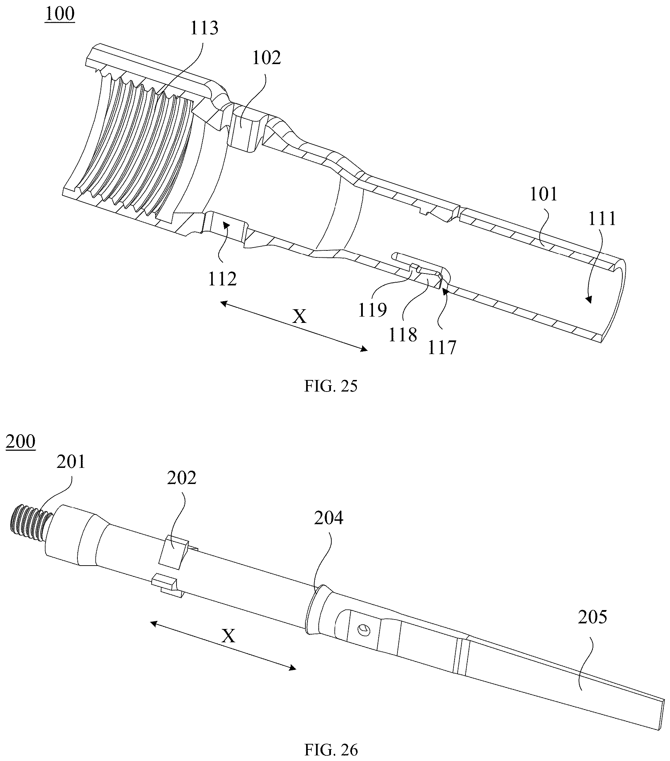

In some other embodiments of the present application, Fig. 25 shows a schematic cross-sectional view of another liquid flow sleeve according to an embodiment of the present application. Referring to Fig. 25, the sleeve body 101 is provided with at least one second mounting hole 117 running through the side wall of the sleeve body 101, and the first mounting part 116 includes an elastic piece 118 and a limiting protrusion 119 connected to each other. The elastic piece 118 is located in the second mounting hole 117, and the limiting protrusion 119 is located inside the mounting cavity 111.

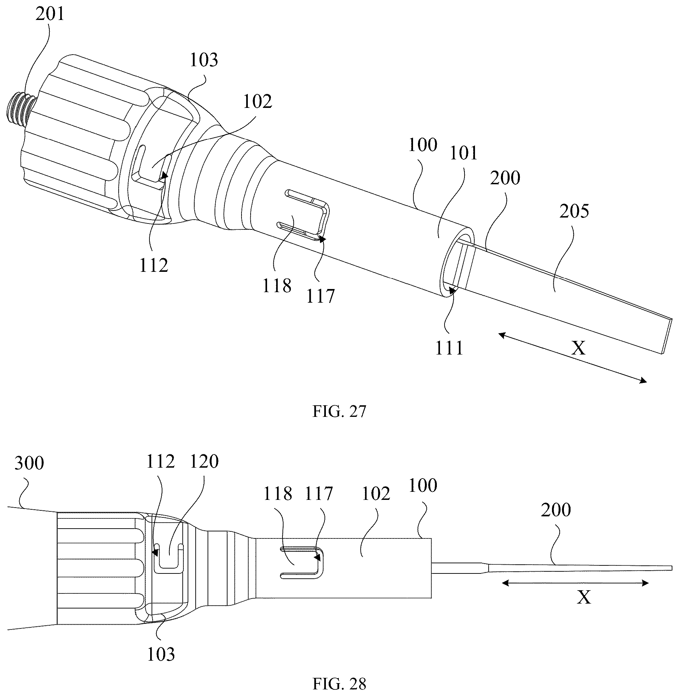

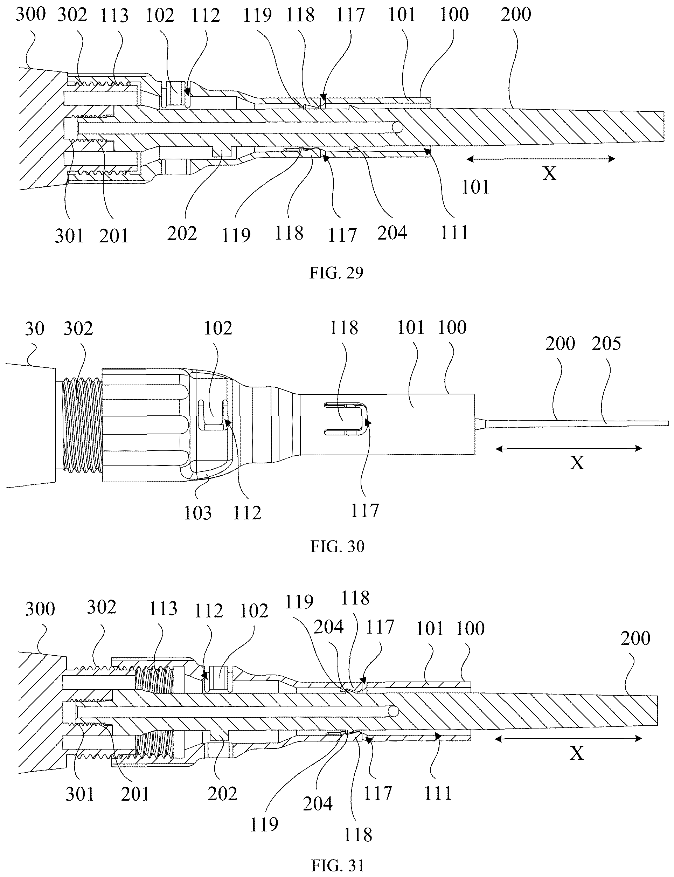

Fig. 26 shows a schematic structural diagram of another blade shank according to an embodiment of the present application. Fig. 27 shows a schematic structural diagram of another medical ultrasonic scalpel according to an embodiment of the present application. Fig. 28 shows a schematic structural diagram of another medical ultrasonic scalpel. Fig. 29 shows a schematic cross-sectional view of the medical ultrasonic scalpel in Fig. 28. Fig. 30 shows a schematic structural diagram of the medical ultrasonic scalpel in another state. Fig. 31 shows a schematic cross-sectional view of the medical ultrasonic scalpel in Fig. 30. Referring to Figs. 26 to 31, when the first mounting part 116 includes the elastic piece 118, the second mounting part 203 includes a boss 204 on an outer side wall surface of the blade shank. The boss 204 abuts against the limiting protrusion 119.

In the embodiments of the present application, through the above structure, during assembly of the medical ultrasonic scalpel, the boss 204 abuts against the limiting protrusion 119 to restrict the movement between the liquid flow sleeve 100 and the blade shank 200. Moreover, the elastic piece 118 also blocks the movement of the boss 204, thereby realizing the connection between the liquid flow sleeve 100 and the blade shank 200. When the medical ultrasonic scalpel is disassembled, the liquid flow sleeve 100 or the blade shank 200 is pushed to cause the boss 204 to be separated from the limiting protrusion 119, thereby realizing the disassembly of the liquid flow sleeve 100 and the blade shank 200.

An embodiment of the present application provides a medical ultrasonic scalpel system, including a vibration source and the medical ultrasonic scalpel according to the above embodiments. The vibration source is connected to the medical ultrasonic scalpel and is configured to generate vibration.

The assembly of the medical ultrasonic scalpel system according to the embodiment of the present application is more convenient.

An embodiment of the present application provides a robot-assisted ultrasonic scalpel system, including a robot-assisted surgical device and the medical ultrasonic scalpel system according to the above embodiment. The robot-assisted surgical device is connected to the medical ultrasonic scalpel of the medical ultrasonic scalpel system to control the movement of the medical ultrasonic scalpel.

A robot-assisted ultrasonic osteodynamic system according to an embodiment of the present application improves the working convenience of orthopedic surgeries.

It should be understood that, in this description, the orientations or positional relationships or dimensions denoted by the terms, such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial" and "circumferential", are the orientations or positional relationships or dimensions shown on the basis of the drawings, and these terms are used merely for ease of description, rather than indicating or implying that the device or element referred to must have particular orientations and be constructed and operated in the particular orientations, and therefore should not be construed as limiting the scope of protection of the present application.

In addition, the terms such as "first", "second" and "third" are merely for descriptive purposes and should not be construed as indicating or implying relative importance or implicitly indicating the number of technical features indicated. Thus, the features defined with "first", "second" and "third" may explicitly or implicitly include one or more features. In the description of the present application, the term "plurality of" means two or more, unless specifically and specifically limited otherwise.

In the present application, unless expressly stated or limited otherwise, the terms such as "mounting", "connection", "connected" and "fixing" should be interpreted broadly, for example, either a fixed or detachable connection, or integration; may be a mechanical connection, or an electrical connection, or communication; and may be a direct connection or an indirect connection by means of an intermediate medium, or may be internal communication between two elements or interaction between the two elements. For those of ordinary skills in the art, the specific meaning of the terms mentioned above in the present application may be construed according to specific circumstances.

In the present application, unless expressly stated or limited otherwise, the expression of the first feature being "above" or "below" the second feature may include the case where the first feature is in direct contact with the second feature, and may also include the case where the first and second features are not in direct contact but are contacted via another feature therebetween. Furthermore, the first feature being "over", "above" or "on" the second feature includes the case where the first feature is directly or obliquely above the second feature, or merely indicates that the first feature is at a higher level than the second feature. The first feature being "below", "under" or "beneath" the second feature includes the case where the first feature is directly or obliquely below the second feature, or merely indicates that the first feature is at a lower level than the second feature.

This description provides many different embodiments or examples that can be used to implement the present application. It should be understood that these various embodiments or examples are purely illustrative and are not intended to limit the scope of protection of the present application in any way. On the basis of the disclosure of the description of the present application, those skilled in the art will be able to conceive of various changes or substitutions. Any changes or substitutions shall fall within the scope of protection of the present application. Therefore, the scope of protection of the present application shall be subject to the scope of protection of the claims.

## Claims

1. A medical ultrasonic scalpel, comprising:
a liquid flow sleeve (100), comprising a sleeve body (101) and a first protruding part (102), wherein the sleeve body (101) is provided with a mounting cavity (111) running through two opposite ends of the sleeve body (101), and the first protruding part (102) is located on an inner side wall of the sleeve body (101); and
a blade shank (200), wherein an extension direction of the blade shank (200) is the same as an extension direction X of the liquid flow sleeve (100), the blade shank (200) runs through the mounting cavity (111), the blade shank (200) is pivotally connected to the liquid flow sleeve (100), the blade shank (200) and the liquid flow sleeve (100) are movable in the extension direction X, and an outer side wall of the blade shank (200) is provided with a second protruding part (202),
wherein the first protruding part (102) and the second protruding part (202) are configured to cooperate such that when the liquid flow sleeve (100) rotates in a first rotation direction, the first protruding part (102) and the second protruding part (202) are offset from each other, so as to enable the liquid flow sleeve (100) to move in the extension direction X, and when the liquid flow sleeve (100) rotates in a second rotation direction opposite to the first rotation direction, the first protruding part (102) abuts against the second protruding part (202), so as to drive the blade shank (200) to rotate in the second rotation direction.

2. The medical ultrasonic scalpel according to claim 1, wherein the sleeve body (101) is provided with at least one first mounting hole (112) running through a side wall of the sleeve body (101); and
the first protruding part (102) comprises a first portion (121) and a second portion (122) connected to each other, the first portion (121) is located in the first mounting hole (112) and connected to the sleeve body (101), the second portion (122) is located in the mounting cavity (111), and the second portion (122) and the second protruding part (202) are configured such that, when the liquid flow sleeve (100) rotates in the first rotation direction, the second protruding part (202) pushes the second portion (122) to move into the first mounting hole (112), and when the liquid flow sleeve (100) rotates in the second rotation direction, the second portion (122) abuts against the second protruding part (202).

3. The medical ultrasonic scalpel according to claim 2, wherein the second portion (122) comprises a first surface (1221) and a second surface (1222), the first surface (1221) and the second surface (1222) being both parallel to the extension direction X,
wherein the first surface (1221) is configured such that, when a thrust applied to the first surface (1221) toward the second surface (1222) is greater than a first preset thrust value, the second portion (122) is moved into the first mounting hole (112), and the second surface (1222) is configured such that, when a thrust applied to the second surface (1222) toward the first surface (1221) is greater than a second preset thrust value, the liquid flow sleeve (100) is moved.

4. The medical ultrasonic scalpel according to claim 3, wherein the second protruding part (202) comprises a third surface (221) and a fourth surface (222), the third surface (221) and the fourth surface (222) being both parallel to the extension direction X;
when the liquid flow sleeve (100) rotates in the first rotation direction, the third surface (221) is configured to apply a thrust to the first surface (1221) toward the second surface (1222); and
when the liquid flow sleeve (100) rotates in the second rotation direction, the fourth surface (222) is configured to apply a thrust to the second surface (1222) toward the first surface (1221).

5. The medical ultrasonic scalpel according to claim 3, wherein a central axis of rotation of the liquid flow sleeve (100) is located on a plane where the second surface (1222) is located, and the central axis of rotation of the liquid flow sleeve (100) is offset from a plane where the first surface (1221) is located.

6. The medical ultrasonic scalpel according to any one of claims 2 to 5, wherein the sleeve body (101) is provided with two first mounting holes (112), the two first mounting holes (112) being symmetrically spaced apart in a circumferential direction of the side wall of the sleeve body (101), and the liquid flow sleeve (100) comprises two first protruding parts (102) in a one-to-one correspondence with the two first mounting holes (112).

7. The medical ultrasonic scalpel according to any one of claims 2 to 5, wherein the liquid flow sleeve (100) further comprises:
a protrusion (103) connected to an outer side wall of the sleeve body (101), the protrusion (103) being arranged spaced apart from the first mounting hole (112).

8. The medical ultrasonic scalpel according to any one of claims 1 to 5, wherein the sleeve body (101) is provided with a first mounting part (116), and the blade shank (200) is provided with a second mounting part (203) connected to the first mounting part (116).

9. The medical ultrasonic scalpel according to claim 8, wherein the first mounting part (116) comprises a first thread located on the side wall of the sleeve body (101), and the second mounting part (203) comprises a second thread located on a side wall of the blade shank (200), the second thread being threadedly connected to the first thread.

10. The medical ultrasonic scalpel according to claim 8, wherein the sleeve body (101) is provided with at least one second mounting hole (117) extending through the side wall of the sleeve body (101), and the first mounting part (116) comprises an elastic piece (118) and a limiting protrusion (119) connected to each other, the elastic piece (118) being located in the second mounting hole (117), and the limiting protrusion (119) being located in the mounting cavity (111); and
the second mounting part (203) comprises a boss (204) located on an outer side wall surface of the blade shank (200), the boss (204) abutting against the limiting protrusion (119).

11. The medical ultrasonic scalpel according to any one of claims 1 to 5, wherein one end of the blade shank (200) is provided with a third mounting part (201), one end of the sleeve body (101) is provided with a fourth mounting part (113), and the medical ultrasonic scalpel further comprises:
a handle (300),
wherein the handle (300) is provided with a fifth mounting part (301) connected to the third mounting part (201), and a sixth mounting part (302) connected to the fourth mounting part (113), the fifth mounting part (301) and the sixth mounting part (302) being both located at an end of the handle (300) facing the blade shank (200).

12. The medical ultrasonic scalpel according to claim 11, wherein the third mounting part (201) comprises a third thread located on a side wall of the blade shank (200), and the fifth mounting part (301) comprises a fourth thread located on a side wall of the handle (300), the fourth thread being threadedly connected to the third thread.

13. The medical ultrasonic scalpel according to claim 11, wherein the fourth mounting part (113) comprises a fifth thread located on the side wall of the sleeve body (101), and the sixth mounting part (302) comprises a sixth thread located on a side wall of the handle (300), the sixth thread being threadedly connected to the fifth thread.

14. The medical ultrasonic scalpel according to claim 11, wherein the inner side wall of the sleeve body (101) is provided with a first mounting groove (114), and the fourth mounting part (113) comprises an elastic ring (115), an outer ring of the elastic ring (115) is fixedly connected to the first mounting groove (114), and a spacing between an inner ring and the outer ring of the elastic ring (115) is greater than a depth of the first mounting groove (114); and
an outer side wall of the end of the handle (300) facing the blade shank (200) is provided with a second mounting groove (303) configured to accommodate the elastic ring (115).

15. A medical ultrasonic scalpel system, comprising a vibration source, and a medical ultrasonic scalpel according to any one of claims 1 to 14,
wherein the vibration source is connected to the medical ultrasonic scalpel and is configured to generate vibration.

16. A robot-assisted ultrasonic scalpel system, comprising a robot-assisted surgical device, and a medical ultrasonic scalpel system according to claim 15,
wherein the robot-assisted surgical device is connected to the medical ultrasonic scalpel of the medical ultrasonic scalpel system to control the movement of the medical ultrasonic scalpel.
